(19) Europäisches Patentamt
European Patent Office
Office européen des brevets

(11) Publication number : **0 494 754 A1**

# (12) EUROPEAN PATENT APPLICATION

(21) Application number : **92300120.0**

(22) Date of filing : **07.01.92**

(51) Int. Cl.[5] : **C07D 213/69,** A61K 31/44

(30) Priority : **09.01.91 GB 9100465**

(43) Date of publication of application : **15.07.92 Bulletin 92/29**

(84) Designated Contracting States : **AT BE CH DE DK ES FR GB GR IT LI LU MC NL PT SE**

(71) Applicant : **Hider, Robert Charles**
**257 Point Clear Road**
**St. Osyth, Clacton, Essex CO16 8JL (GB)**
(71) Applicant : **Peto, Timothy Edward Alexander**
**79 Lonsdale Road**
**Oxford, OX2 7ES (GB)**
(71) Applicant : **Whitehead, Susan**
**Calver Hey, 6 Bermuda Avenue**
**Skirlaugh, Hull HU11 5HG (GB)**

(72) Inventor : **Hider, Robert Charles**
**257 Point Clear Road**
**St. Osyth, Clacton, Essex CO16 8JL (GB)**
Inventor : **Peto, Timothy Edward Alexander**
**79 Lonsdale Road**
**Oxford, OX2 7ES (GB)**
Inventor : **Whitehead, Susan**
**Calver Hey, 6 Bermuda Avenue**
**Skirlaugh, Hull HU11 5HG (GB)**

(74) Representative : **Stephenson, Gerald Frederick**
**Patents Department, British Technology Group plc, 101 Newington Causeway**
**London SE1 6BU (GB)**

(54) **3-hydroxypyridin-4-one derivatives as pharmaceutical compositions.**

(57) 3-Hydroxypyridin-4-ones of formula (I)

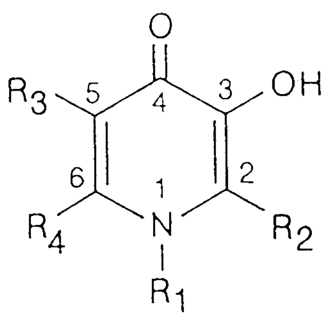

in which $R_1$ is selected from hydrogen, $C_{1-3}$ aliphatic hydrocarbon groups, 2-hydroxyethyl, $C_{1-4}$ aliphatic hydrocarbon groups substituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, and $R_2$, $R_3$ and $R_4$ are each separately selected from hydrogen, $C_{1-3}$ aliphatic hydrocarbon groups, hydroxymethyl, 1-hydroxyethyl, 2-hydroxyethyl, 2-methoxymethyl, $C_{1-4}$ aliphatic hydrocarbon groups substituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, but with the provisos firstly that one of $R_1$ to $R_4$ is a $C_{1-4}$ aliphatic hydrocarbon group substituted by a single carboxy, sulpho, sulphamoyl or N-methyl or N-ethyl sulphamoyl group, or an ethyl group substituted at the 1- or 2-position by a carboxy, sulpho, sulphamoyl or N-methyl or N-ethyl sulphamoyl group and at the 2-position by a hydroxy group, secondly that either $R_2$ or $R_3$ is a group other than hydrogen and thirdly that the total number of atoms other than hydrogen present in $R_1$ to $R_4$ is no more than eight, the compound optionally being in the form of a physiologically acceptable salt and/or pro-drug thereof, are of value for the treatment of conditions caused by iron dependent parasites, particularly malaria.

EP 0 494 754 A1

Jouve, 18, rue Saint-Denis, 75001 PARIS

This invention relates to the treatment of parasitic infections and in particular to the treatment of malaria and other conditions in which the parasite is iron dependent.

Malaria parasites (Plasmodium spp.) show a complex pattern of development in the mammalian host. Infection is initiated when female mosquitoes feeding on blood inoculate sporozoites into the bloodstream from whence they enter the liver. The parasites proliferate in the liver and are ultimately released therefrom in the mature form, known as merozoites, which initiate the asexual erythrocytic cycle which causes the disease. The parasites, once taken up as merozoites by the red blood cells, pass through various stages, including the trophozoite and schizont stages, which culminate in the rupture of the blood cells with the release of much increased numbers of merozoites, thereby perpetuating the cycle.

Malaria presents a very considerable medical problem, despite the development of various drugs for its treatment. This is particularly the case since, of the different species of Plasmodium responsible for malaria in humans, P. falciparum is the species which causes the most morbidity and mortality and is at the same time the one which poses most problems by way of resistance to the preferred therapeutic agent, chloroquine.

It is known that iron chelators will exert an anti-malarial effect through interaction with the intracellular low molecular weight iron pool, the ability of the drug to cross an infected cell membrane therefore being a prerequisite for anti-malarial activity. Desferrioxamine has been used an an anti-malarial drug but this iron chelator has the disadvantage of being relatively toxic and having to be administered parenterally because it is poorly absorbed from the gastrointestinal tract and is also rapidly removed from the blood.

A recent paper by Heppner et al, Blood, 1988, 72, 358, discusses the use of 3-hydroxypyridin-4-one iron chelators as anti-malarial drugs. The compounds described therein are all neutral under physiological conditions, containing alkyl group substituents at the 1- and 2-positions of the ring. 3-Hydroxypyridin-4-ones containing substituents which are charged are not regarded as being of particular value for the treatment of iron overload (Porter et al, Blood, 1988, 72, 1497) and it would also be expected that they would be of little value as anti-malarial drugs since their charge would be expected to prevent them from crossing the infected cell membrane. We have now found, however, that a particular group of charged 3-hydroxypyridin-4-ones is of especial value as anti-malarial drugs. Thus, these compounds have been found to possess the unexpected property of showing a selective toxic effect on malaria infested erythrocytes.

Accordingly the present invention comprises the use for the manufacture of a medicament for use in the treatment of a condition caused by an iron dependent parasite of a 3-hydroxypyridin-4-one of formula (I)

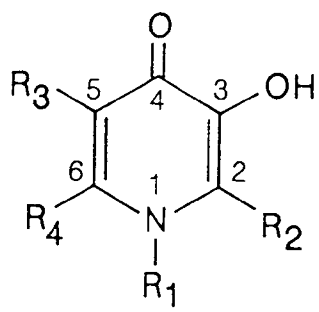

(I)

in which $R_1$ is selected from hydrogen, $C_{1-3}$ aliphatic hydrocarbon groups, 2-hydroxyethyl, $C_{1-4}$ aliphatic hydrocarbon groups substituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, and $R_2$, $R_3$ and $R_4$ are each separately selected from hydrogen, $C_{1-3}$ aliphatic hydrocarbon groups, hydroxymethyl, 1-hydroxyethyl, 2-hydroxyethyl, methoxymethyl, $C_{1-4}$ aliphatic hydrocarbon groups substituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, but with the provisos firstly that one of $R_1$ to $R_4$ is a $C_{1-4}$ aliphatic hydrocarbon group substituted by a single carboxy, sulpho, sulphamoyl or N-methyl or N-ethyl sulphamoyl group, or an ethyl group substituted at the 1- or 2-position by a carboxy, sulpho, sulphamoyl or N-methyl or N-ethyl sulphamoyl group and at the 2-position by a hydroxy group, secondly that either $R_2$ or $R_3$ is a group other than hydrogen, and thirdly that the total number of atoms other than hydrogen present in $R_1$ to $R_4$ is no more than eight, the compound optionally being in the form of a physiologically acceptable salt and/or pro-drug thereof.

The particular value of the compounds (I) of use in the present invention arises from the contrast between their effect on parasite infected cells, particularly parasite infected erythrocytes, and their effect on other cells. Thus we have found, using a murine erythroleukaemia (MEL) cell line as a model for such other cells, that the compounds (I) show a similar level of activity against malaria infected parasites to desferrioxamine and alkyl substituted 3-hydroxypyridin-4-ones such as those described by Heppner et al, ibid, but in contrast to those compounds the compounds (I) do not have a significant growth inhibiting effect on the MEL cell line. This means that the compounds (I) may be used at the quite high dosage levels required for the treatment of parasitic infections without incurring the significant level of toxic side effects experienced with compounds which do not have a selective action.

The term aliphatic hydrocarbon group is used herein in relation to both unsubstituted and carboxy-substituted groups to include both acyclic and cyclic groups which may be unsaturated or saturated, the acyclic groups having a branched chain or especially a straight chain. Whilst the cyclic aliphatic hydrocarbon groups are preferably saturated, for example being cyclopropyl, both saturated and unsaturated groups are of interest in the case of the acyclic groups, for example allyl and propargyl as well as methyl, ethyl, propyl and isopropyl. Butyl and its branched chain analogues may also be mentioned in relation to the substituted aliphatic hydrocarbon groups but in general it is $C_{1-3}$ acyclic groups and $C_3$ cyclic groups which are of more interest in this case.

As regards $R_1$, although this may be hydrogen there is greater interest in the other groups, particularly the $C_{1-3}$ aliphatic hydrocarbon groups and the substituted $C_{1-4}$ aliphatic hydrocarbon groups. As regards $R_2$, $R_3$ and $R_4$, although these may be 1-hydroxyethyl, 2-hydroxyethyl, especially methoxymethyl and particularly hydroxymethyl, there is greater interest in the other groups, particularly hydrogen, the $C_{1-3}$ aliphatic hydrocarbon groups and the substituted $C_{1-4}$ aliphatic hydrocarbon groups.

Only one of the groups $R_1$ to $R_4$ contains a carboxy, sulpho, sulphamoyl or N-methyl or N-ethyl sulphamoyl group, and it is preferred that this group is carboxy rather than a sulpho, sulphamoyl or N-methyl or N-ethyl sulphamoyl group, although if one of such latter groups is present it is preferably a sulphamoyl or, to a lesser extent, substituted sulphamoyl group rather than a sulpho group. The preference for which of the four groups is this one group follows the order $R_1 > R_2 > R_4 > R_3$ so that the particular preference is for $R_1$ to be this group. Although the group may be an ethyl group substituted by a hydroxy and a sulpho, sulphamoyl or N-methyl or N-ethyl sulphamoyl group, or particularly a carboxy group, for example $-CH_2CH(OH)CO_2H$, such groups are less preferred. Instead, the group substituted by a carboxy-, sulpho, sulphamoyl or N-methyl or N-ethyl sulphamoyl group, is conveniently a $C_{1-4}$ aliphatic hydrocarbon group with a single carboxy, sulpho, sulphamoyl or N-methyl or N-ethyl sulphamoyl group substituent, preferably a substituted $C_{1-3}$ aliphatic hydrocarbon group, particularly a substituted alkyl group and especially, in increasing order of preference, a substituted isopropyl, propyl or ethyl group, and the group is conveniently terminally substituted. As regards the other groups of $R_1$ to $R_4$ these are most conveniently selected from ethyl and especially methyl, and, in the case of $R_2$, $R_3$ and $R_4$ also from hydrogen. It is preferred that $R_4$ is hydrogen and also that only one of $R_2$ and $R_3$ is other than hydrogen, the group $R_3$ conveniently being that which is hydrogen.

The total number of carbon, nitrogen, oxygen and sulphur atoms present in $R_1$ to $R_4$ includes the three atoms of the carboxy group, the four atoms of the sulpho or sulphamoyl group, the five atoms of the N-methyl sulphamoyl group and the six atoms of the N-ethyl sulphamoyl group, and is no more than eight. Preferably, however, it is no more than seven. Although the minimum number of carbon, nitrogen, oxygen and sulphur atoms which may be present in $R_1$ to $R_4$ is four, corresponding to the case where $R_1$ and $R_4$ are each hydrogen and one of $R_2$ and $R_3$ is also hydrogen with the other being a carboxymethyl group, the preferred minimum is five atoms. The preferred value for the total number of carbon, nitrogen, oxygen and sulphur atoms in $R_1$ to $R_4$ is thus five to seven, for example six.

The 3-hydroxypyridin-4-ones of particular interest contain one ethyl or especially methyl substituent, for example at the 2-position, and one substituent which is 2-carboxy-2-hydroxyethyl, 2-sulphamoylethyl, 2-N-methylsulphamoylethyl or 2-N-ethylsulphamoylethyl or particularly, in increasing order of preference, 1-carboxyethyl, carboxymethyl, 2-carboxypropyl, 3-carboxypropyl or 2-carboxyethyl, for example at the 1-position. Specific compounds (I) of interest are thus:

(1) 1-(2-carboxy-2-hydroxyethyl)-2-methylpyridin-4-one;
(2) 3-hydroxy-2-methyl-1-(2-sulphamoylethyl)pyridin-4-one;
(3) 3-hydroxy-2-methyl-1-(2-N-methylsulphamoylethyl)pyridin-4-one;
(4) 1-(2-N-ethylsulphamoylethyl)-3-hydroxy-2-methylpyridin-4-one;
(5) 2-carboxymethyl-3-hydroxy-1-methylpyridin-4-one;
(6) 2-carboxymethyl-1-ethyl-3-hydroxypyridin-4-one;
(7) 2-(2-carboxyethyl)-3-hydroxy-1-methylpyridin-4-one;
(8) 2-(2-carboxyethyl)-1-ethyl-3-hydroxypyridin-4-one;

(9) 1-carboxymethyl-3-hydroxy-2-methylpyridin-4-one;
(10) 1-carboxymethyl-2-ethyl-3-hydroxypyridin-4-one;
(11) 1-(2-carboxyethyl)-3-hydroxy-2-methylpyridin-4-one;
(12) 1-(2-carboxyethyl)-2-ethyl-3-hydroxypyridin-4-one;
(13) 1-(1-carboxyethyl)-2-methyl-3-hydroxypyridin-4-one;
(14) 1-(1-carboxyethyl)-2-ethyl-3-hydroxypyridin-4-one;
(15) 1-(2-carboxypropyl)-2-methyl-3-hydroxypyridin-4-one; and
(16) 1-(3-carboxypropyl)-2-methyl-3-hydroxypyridin-4-one;

the compounds (2) to (4), especially (2), and (9) to (12) and (16), especially (11), being of most interest.

The most preferred compound is compound (11), this having shown a higher level of activity against red blood cells parasitized by P. falciparum than compounds (12) and (16), which in turn have shown a higher level of activity than compound (9), the activity of which is somewhat greater than that of the compound 3-hydroxy-2-methyl-1-(2-sulphoethyl)pyridin-4-one.

It will be appreciated that certain of the compounds (I) are novel per se and are included within the scope of the present invention.

The compounds may, if desired, be used in the form of salts of several types. Thus, when not in salt form, the compounds of use in the present invention can exist in an uncharged form but will usually exist predominantly in a zwitterionic form. However they can also exist as salts in both a cationic form and in two anionic forms. The various forms are illustrated below for the compound 1-(2-carboxyethyl)-3-hydroxy-2-methylpyridin-4-one, formulae (Ia), (II), (III), (IV) and (V) showing respectively the uncharged, zwitterionic, cationic, and monovalent and divalent anionic forms

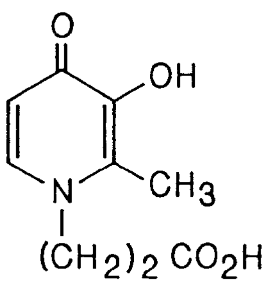

(Ia)

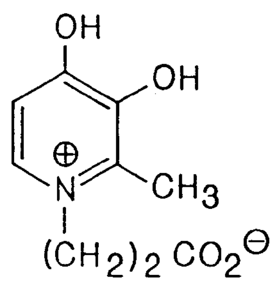

(II)

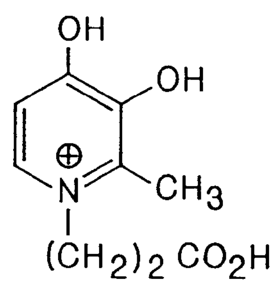

(III)

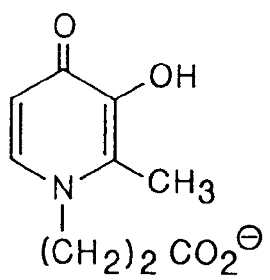

(IV)

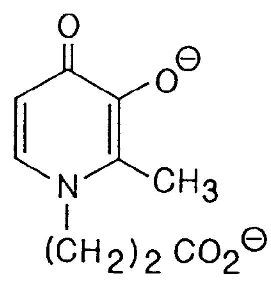

(V)

It is, therefore, possible to form salts with both physiologically acceptable bases and acids, the former containing one of the anionic forms exemplified in (IV) and (V) and the latter containing the cationic form exemplified in (III). Examples of suitable bases are the alkali metal hydroxides, for example sodium hydroxide, quaternary ammonium hydroxides and amines such as tris (tris representing 2-amino-2-hydroxymethyl propane 1,3-diol). Suitable acids may be inorganic or organic. Examples of such inorganic acids are phosphoric acid, nitric acid, sulphuric acid and particularly the hydrohalic acids hydrochloric acid, hydrobromic acid and hydroiodic acid. Examples of such organic acids are citric acid, oxalic acid, fumaric acid, maleic acid, lactic acid, succinic acid, malic acid, tartaric acid and methane sulphonic acid.

Alternatively, or additionally, the compounds may be in the form of a pro-drug in which the 3-hydroxy group is in the form of another group which is reconverted thereto in vivo. Such a pro-drug group may, for example, be as described in U.S. Patent 4,908,371 and its equivalents European Patent Application 0316279A and

Japanese Patent Application 89/157962, in particular being a group R′COO wherein R′ is a $C_{1-8}$ alkyl group, especially a $C_{3-7}$ group which is branched at the carbon atom adjacent to the carbonyl group, such as an isopropyl or t-butyl group. Additionally, or alternatively, the carboxy group may be in pro-drug form, for example as an ester. Such esters may be formed with both phenols and alcohols, the latter being of particular interest and including alkanols, especially those of one to six carbon atoms such as methanol, ethanol and the propanols and butanols. However, in the case of both types of pro-drug any group is suitable which is removed in vivo to regenerate the hydroxy or carboxy group, particularly after the compound has entered the bloodstream.

The compounds (I) may be prepared by procedures such as those described in U.K. Patent GB 2,136,807B and by variations thereon which will be apparent to those skilled in the art. In particular a corresponding 3-hydroxy-4-pyrone, or a 3-hydroxy-4-pyrone containing groups convertible to the C-substituents present in the desired hydroxypyridinone can be reacted with a compound $R'NH_2$ in which R′ represents the group present on the nitrogen atom of the desired compound or a group convertible thereto, the reaction being carried out in the presence of a base, for example an alkali metal hydroxide such as sodium hydroxide. This procedure is specifically exemplified in Examples 16 and 17 of GB 2136807B for the preparation from maltol of 1-carboxymethyl- and 1-(2′-carboxyethyl)-3-hydroxy-2-methyl-pyridin-4-one and may be applied in an exactly analogous fashion to the preparation of N-aliphatic hydrocarbyl compounds.

Alternative 3-hydroxy-4-pyrone starting materials to maltol are readily available, for example as described in published U.S. Patent Application Serial No. 310,141 (series of 1960) or otherwise. Thus, for example, the compound 3-hydroxy-2-hydroxymethyl-4-pyrone may be converted to the corresponding 2-carboxymethyl compound through protection of the 3-hydroxy group, for example by benzylation, bromination of the remaining hydroxy group, formation of a Grignard reagent, reaction thereof with carbon dioxide and deprotection to provide 2-carboxymethyl-3-hydroxy-4-pyrone. This compound may be used for the preparation of various 1-aliphatic hydrocarbyl-2-carboxymethyl-3-hydroxypyridin-4-ones. Alternatively, 2-carboxymethyl-3-hydroxy-4-pyrone may be prepared by the oxidation of 3-hydroxy-2-hydroxyethyl-4-pyrone. Similar procedures may be used for preparing pyrones containing other 2-carboxyalkyl substituents.

An alternative general route involves nucleophilic substitution at the nitrogen atom of the corresponding 3,4-dihydroxypyridine (or 3-hydroxypyridin-4-one) or of such a compound containing groups convertible to the C-substituents present in the desired hydroxypyridinone, for example using an organic halide R′X in which R′ represents the group present on the nitrogen atom of the desired compound or a group convertible thereto. Yet another route involves reaction of the corresponding 3-hydroxypyridin-4-one or one containing groups convertible to the C-substituents present in the desired hydroxypyridinone with an amine $R'NH_2$ in which R′ represents the group present on the nitrogen atom of the desired compound or a group convertible thereto.

In most cases it will be appropriate to protect the 3-hydroxy group in the reactant hydroxypyrone or hydroxypyridinone precursor A common form of protection is to convert the hydroxy group to a benzyloxy group which can eventually be removed by catalytic hydrogenation. Moreover, as indicated it may be appropriate for one or more substituent groups in the initially formed 3-hydroxypyridin-4-one to be modified to provide the desired substituents.

Salts may readily be formed by reaction of the compound (I) with the appropriate base or acid under suitable conditions. Thus, the zwitterionic form may conveniently be obtained by freeze drying an aqueous solution at a pH of about 4.0 but freeze drying of an aqueous solution whose pH has been adjusted to 7.0 or to greater than 9.0 with the desired base provides a convenient route to a salt of that base containing an anion of the type (IV) or (V) respectively. Salts with acids may conveniently be obtained by recrystallization of the compound (I) from an aqueous/organic solution, for example the hydrochloride being obtained on recrystallization from a dilute hydrochloric acid/ethanol solution.

Pro-drugs may be formed by reaction of the compound (I) or a derivative thereof with the appropriate reagent, particularly with an organic acid or derivative thereof, for example as described in U.S. Patent 4,908,371 and/or with an alcohol or phenol, for example using standard esterification procedures.

The compounds (I) may be formulated with a physiologically acceptable diluent or carrier for use as pharmaceuticals for veterinary, for example in a mammalian context, and particularly for human use by a variety of methods. For instance, they may be applied as a composition incorporating a liquid diluent or carrier, for example an aqueous or oily solution, suspension or emulsion, which may often be employed in injectable form for parenteral administration and therefore may conveniently be sterile and pyrogen free. Oral administration may also be used, and indeed is preferred. Although compositions for this purpose may incorporate a liquid diluent or carrier, it is more usual to use a solid, for example a conventional solid carrier material such as starch, lactose, dextrin or magnesium stearate. Such solid compositions may conveniently be of a formed type, for example as tablets, capsules (including spansules), etc.

Other forms of administration than by injection or through the oral route may also be considered in both

human and veterinary contexts, for example the use of suppositories or pessaries. Another form of pharamceutical composition is one for buccal or nasal administration, for example lozenges, nose drops or an aerosol spray.

Thus, the invention further includes a pharmaceutical composition comprising a 3-hydroxypyridin-4-one of formula (I) as defined hereinbefore together with a physiologically acceptable diluent or carrier.

Compositions may be formulated in unit dosage form, i.e. in the form of discrete portions each comprising a unit dose, or a multiple or sub-multiple of a unit dose. Whilst the dosage of active compound given will depend on various factors, including the particular compound which is employed in the composition and the mode of administration and type of parasitic infection to be treated, it may be stated by way of guidance that satisfactory control of an iron dependent parasitic infection in the human body will often be achieved by maintaining a concentration of the compound in the bloodstream which provides a 10-50 μM iron binding capacity (3 moles of the compound binding with 1 mole of iron). A dosage appropriate to provide such an iron binding capacity will usually lie in the region of about 0.5 or 1 g to 15 or 20 g daily, particularly of about 1 or 2 g to 10 or 15 g daily, for example about 5 g, veterinary doses being on a similar g/kg body weight ratio. However, it will be appreciated that it may be appropriate under certain circumstances to give daily dosages either below or above these levels, particularly with the more active compounds such as 1-(2-carboxyethyl)-3-hydroxy-2-methylpyridin-4-one. Where desired, more than one compound according to the present invention may be administered in the pharmaceutical composition, when the total dosage will usually correspond to those discussed above, or, indeed, other active compounds may be included in the composition.

The compounds are of interest for the treatment of any condition caused by an iron dependent parasite, particularly those which infect erythrocytes. Such parasites include malaria and in particular those species of Plasmodium which infect the human such as P. knowlesii and also P. vivax, P. ovale and P. malariae but particularly P. falciparum. Other parasites which may be specifically mentioned are the causative agents of babesiosis, which are various species of Babesia such as B. divergens, and of tryponosomiasis, which are various species of Trypanosoma such as T. croezi.

It will be appreciated that the compounds may well be more effective against the parasite at one particular stage of its cell cycle than at others, as indeed is found to be the case with malaria where it has been found that control of the parasite is maximal at the late trophozoite and early schizont stages of the development of the parasite. However, since the life cycle of the parasite is usually relatively short, for example about 48 hours for P. falciparum in the human, then it is simplest to continue the period of treatment so that a suitable concentration of the compound in the bloodstream, as discussed hereinbefore, is maintained at least for the duration of the parasite cell cycle and conveniently for about 4 or 5 such cycles, i.e. for a period of about 7 to 10 days. This will usually require a daily dosage as indicated hereinbefore although it will be appreciated that for compounds with a long half life it may be possible to reduce the frequency of administration of the compound from a daily dosage.

The present invention therefore includes a method for the treatment of a patient having a condition caused by an iron dependent parasite, for example malaria, which comprises administering to that patient a therapeutically effective amount of a compound of formula (I) as defined hereinbefore.

The previously discussed inefficiency of compounds containing charged substituents at reducing overall levels of iron in the body is of course an advantage in the context of the present invention where the aim is the treatment of malaria in patients who will not generally be suffering from iron overload.

The invention is illustrated by the following Examples.

## EXAMPLES

### Example 1 : Preparation of 1-(3-carboxpropyl)-3-hydroxy-2-methylpyridin-4-one hydrochloride

(1) 3-Benzyloxy-1-(3-carboxypropyl)-2-methylpyridin-4-one

To a suspension of 3-benzyloxy-2-methyl-4-pyrone (30 g, 0.139 mol, prepared as described in Example 10 of GB 2136807B) in ethanol (150 ml)/water (450 ml) was added 4-aminobutyric acid (14.3 g, 0.139 mol) followed by sodium hydroxide (as ION solution) until the mixture reached pH 13. After stirring at room temperature for 24 hours, concentrated hydrochloric acid was added until the solution reached pH 4, when it was extracted into dichloromethane (2 x 500 ml). The organic fractions were combined, dried over anhydrous sodium sulphate, filtered and rotary evaporated to yield a brown oil. The brown oil was crystallized from acetone to give the title compound (21.2 g, 51%) as pale orange needles, m.p. 169-170°C;
δ(90 MHz; $d_6$DMSO): 1.80 (q, 2H), 2.20 (s, 3H), 2.25 (t, 2H), 3.90 (t, 2H), 5.05 (s, 2H), 6.20 (d, 1H), 7.40 (m, 5H), 7.60 (d, 1H).

(2) 1-(3-Carboxypropyl)-3-hydroxy-2-methylpiridin-4-one hydrochloride

3-Benzyloxy-1-(3-carboxypropyl)-2-methylpyridin-4-one (10 g, 0.0332 mol) was dissolved in ethanol (150 ml)/water (150 ml), the solution adjusted to pH 1 with concentrated hydrochloric acid, and then hydrogenated using a palladium on charcoal catalyst. Filtration followed by rotary evaporation yielded a cream solid which was recrystallized from ethanol/diethyl ether to give the title hydrochloride (7.03 g, 85%) as a white powder, m.p. 226-227°C; δ(90 MHz; $d_6$DMSO): 2.00 (q, 2H), 2.40 (t, 2H),
2.60 (s, 3H), 4.40 (t, 2H), 7.45 (d, 1H), 8.35 (d, 1H).

Note The corresponding 1-carboxymethyl and 1-(2-carboxyethyl) hydrochlorides were prepared by an analogous procedure, the compounds having the melting points 264-266°C and 212-214°C respectively.

Example 2 : Preparation of 1-(2-carboxyethyl)-2-ethyl-3-hydroxypyridin-4-one

To a suspension of 3-benzyloxy-2-ethyl-4-pyrone [20 g, 0.0869 mol; prepared in an analogous procedure to that described for the compound of Example 1(1)] in ethanol (100 ml)/ water (300 ml) was added β-alanine (7.73 g, 0.0869 mol) followed by sodium hydroxide (as ION solution) until the mixture reached pH 13. After stirring at room temperature for 24 hours, concentrated hydrochloric acid was added until the solution reached pH 4, when it was extracted into dichloromethane (2 x 500 ml). The organic fractions were combined, dried over anhydrous sodium sulphate, filtered and rotary evaporated to yield 3-benzyloxy-1-(2-carboxyethyl)-2-ethylpyridin-4-one as a brown oil (10.7 g). The brown oil was dissolved in ethanol (150 ml)/water (150 ml) and the solution was adjusted to pH 1 with concentrated hydrochloric acid and then hydrogenated using a palladium on charcoal catalyst. Filtration followed by rotary evaporation gave an orange oil, which was dissolved in water, and the solution was adjusted to pH 4 with 10% aqueous sodium hydroxide, then boiled with activated charcoal for 15 minutes. Filtration followed by crystallization from the filtrate gave the title compound (2.11 g, 11%) as a white powder, m.p. 162-164°C; δ(90 MHz; $d_6$DMSO): 1.15 (t, 3H), 2.75 (m, 4H), 4.20 (t, 2H), 6.20 (d, 1HH), 6.35 (broad, 2H), 7.65 (d, 1H).

Example 3 : Preparation of 3-hydroxy-2-methyl-1-(2-sulphoethyl)-pyridin-4-one

(1) 3-Benzyloxy-2-methyl-1-(2-sulphoethyl)-pyridin-4-one hydrochloride

To a suspension of 3-benzyloxy-2-methyl-4-pyrone (2.16 g, 0.01 mol) in ethanol (30 ml) and water (30 ml) was added taurine (2 g, 0.015 mol) followed by sodium hydroxide (ION solution) until the mixture reached pH 13. After refluxing for 12 hours, concentrated hydrochloric acid was added until the solution reached pH 2 when a white solid precipitated. Recrystallization from ethanol/water gave the title hydrochloride (2 g, 56%) as white needles, m.p. 270°C (decomposition); δ(90 MHz; $D_20$ + NaOH);
7.76 (d, 1H), 7.40 (m, 5H), 6.60 (d, 1H), 4.98 (s, 2H), 4.31 (t, 2H), 3.15 (t, 2H), 2.04 (s, 3H).

(2) 3-Hydroxy-2-methyl-1-(2-sulphoethyl)-pyridin-4-one

3-Benzyloxy-2-methyl-1-(2-sulphoethyl)-pyridin-4-one (1.16 g, 0.005 mol) was dissolved in water (30 ml)/ ethanol (30 ml), 5% palladium on charcoal catalyst (0.3 g) was added and the mixture stirred under a constant stream of hydrogen for 4 hours at room temperature. After removal of the used catalyst by filtration, the solution was rotary evaporated to give a cream coloured solid. Crystallization from ethanol-water/diethyl ether gave the title compound (0.58 g, 77%), as a white powder, m.p. 337-339°C;
δ(90 MHz; $D_2O$); 7.75 (d, 1H), 6.54 (d, 1H), 4.49 (t, 2H), 3.36 (t, 2H), 2.48 (s, 3H).

Example 4 : Formulation of medicaments

(A) Tablets of the following composition are prepared:

| | mg/tablet |
|---|---|
| 1-(2-carboxyethyl)-3-hydroxy-2-methylpyridin-4-one (micronised) | 250 |
| 'Avicel' (microcrystalline cellulose) | 38 |
| polyvinylpyrrolidone | 3 |
| alginic acid | 6 |
| magnesium stearate | 3 |

The 3-hydroxypyridin-4-one is mixed with 'Avicel' and polyvinylpyrrolidone is added, dissolved in sufficient industrial methylated spirits (74° OP) to produce a mass suitable for granulating. The mass is granulated through a 20 mesh sieve and the resultant granules are dried at a temperature not exceeding 50°C. The dried granules are passed through a 20 mesh sieve and the alginic acid and magnesium stearate are then added and mixed with the granules. The product is compressed into tablets each weighing 300 mg on 3/8 inch flat bevelled edge divided punches.

(B) Tablets of the following composition are prepared:

| | mg/tablet |
|---|---|
| 1-(2-carboxyethyl)-3-hydroxy-2-methylpyridin-4-one (micronised) | 250 |
| 'Avicel' (microcrystalline cellulose) | 134 |
| polyvinylpyrrolidone | 4 |
| alginic acid | 8 |
| magnesium stearate | 4 |

The tablets are prepared by essentially the same procedure as described in (A) and are compressed at a tablet weight of 400 mg on 7/16 inch flat bevelled edge punches.

(C) Tablets of the following composition are prepared:

| | mg/tablet |
|---|---|
| 1-(2-carboxyethyl)-3-hydroxy-2-methylpyridin-4-one (micronised) | 250 |
| lactose (300 mesh) | 19 |
| maize starch | 15 |
| gelatine | 10 |
| magnesium stearate | 6 |

The 3-hydroxypyridin-4-one is mixed with lactose and half the total quantity of maize starch required, and a 5% solution of gelatine in water is added to the mass. The product is granulated through a 16 mesh sieve, and the resultant granules are dried to constant weight at a temperature not exceeding 50°C. The dried granules are passed through a 20 mesh sieve and mixed with magnesium stearate and the remainder of the maize starch.

The product is compressed at a 300 mg tablet weight on 3/8 inch flat bevelled edge divided punches.

Similar procedures may be followed with other compounds such as those of Examples 1 to 3.

Example 5 : Inhibition of growth of P. falciparum

(A) An uncloned strain of P. falciparum, ITO4, was grown on blood group O red cells using a modification of the method described by Trager et al in Science, 1976, 93, 673. An atmopshere consisting of 1% oxygen, 3% carbon dioxide and 96% nitrogen was maintained throughout the experiments. Red cells were suspended at a hematocrit of 3% in RPMI 1640 that had been supplemented with 10% v/v human serum, 2 g/l glucose, 4 mM NaOH, 25 μg/ml gentamicin, 1.9 mM glutamine and 25 mM HEPES to form complete medium. For experimental purposes the red cells were then further diluted with the same medium to a final hematocrit of 1.5%. Synchronised parasite cultures were obtained using a combination of sorbitol lysis and gelatin flotation, as described respectively by Jensen et al in Am. J. Trop. Med. Hyg., 1978, 27, 1274 and Lambros et al in J. Parasitol., 1979, 65, 418, thus ensuring that only young ring forms remained. The starting parasitemia in each experiment was between 0.1 and 0.5%.

A red cell suspension containing only recently synchronised ring parasites was treated with 0.2 μCi of tritiated hypoxanthine per 100 μl of red cell suspension and one of ten chelating agents as identified in the Table was added in complete medium to the suspension in an amount such as to produce an iron binding concentration of 100 μmol of iron per litre (all of the chelators combine with iron in a ratio of 3 moles of chelator:1 mole of iron apart from the chelator number 10, desferrioxamine, which combines in a 1:1 molar ratio). The test cultures were immediately dispersed in 200 μl aliquots into 96 well microculture plates. Each chelator was tested in three or more wells and the whole experiment was carried out at least twice for each chelator. Control cultures were used in each experiment which contained no iron chelator.

Table : Chelating Agents

| No. | Name | Identification | | | |
|---|---|---|---|---|---|
| | | $R_1$ | $R_2$ | $R_3$ | $R_4$ |
| 1 | 1-(2-carboxyethyl)-3-hydroxy-2-methylpyridin-4-one | $CH_2CH_2CO_2H$ | $CH_3$ | H | H |
| 2 | 3-hydroxy-1,2-dimethylpyridin-4-one | $CH_3$ | $CH_3$ | H | H |
| 3 | 3-hydroxy-2-methylpyridin-4-one | H | $CH_3$ | H | H |
| 4 | 3-hydroxy-1-(2-hydroxyethyl)-2-methylpyridin-4-one | $CH_2CH_2OH$ | $CH_3$ | H | H |
| 5 | 1-(2-aminoethyl)-3-hydroxy-2-methylpyridin-4-one hydrochloride | $CH_2CH_2NH_2HCl$ | $CH_3$ | H | H |
| 6 | 3-hydroxy-1-(2-methoxyethyl)-2-methylpyridin-4-one | $CH_2CH_2OCH_3$ | $CH_3$ | H | H |
| 7 | 1-(5-carboxypentyl)-3-hydroxy-2-methylpyridin-4-one | $(CH_2)_5CO_2H$ | $CH_3$ | H | H |
| 8 | 1-(5-(N-(2-carboxyethyl)-carbamoyl)pentyl)-3-hydroxy-2-methylpyridin-4-one | $(CH_2)_5CONH(CH_2)_2CO_2H$ | $CH_3$ | H | H |
| 9 | 2-ethyl-3-hydroxy-1-methyl-pyridin-4-one | $CH_3$ | $C_2H_5$ | | |
| 10 | Desferrioxamine | - | - | - | - |

The growth of the P. falciparum parasites was assessed both by light microscopy examination of thin films that had been stained with Giemsa and through measurement of tritiated hypoxanthine uptake, the cell cultures being harvested onto glass fibre filters and radioactivity, in terms of beta emission from each well, being measured in a flat bed liquid scintillation counter as described by Potter et al in Phys. Med. Biol., 1986, 31(4), 361.

Figure 1 shows in histogram form the results obtained for the percentage inhibition of growth after 30 hours as compared with the controls and represents the pooled results of at least two experiments with each chelator. The chelators tested consisted of 1-(2′-carboxyethyl)-3-hydroxy-2-methylpyridin-4-one ($R_1$ = $CH_2CH_2CO_2H$, $R_2$ = $CH_3$, $R_3$ = $R_4$ = H; compound 1) and, for the purposes of comparison with this chelator, nine other chelators not of formula (I) as defined hereinbefore, of which compounds 2 to 9 are 3-hydroxypyridin-4-ones and compound 10 is desferrioxamine. It will be seen that compounds 1, 2, 3, 4, 5, 6, 9 and 10 each inhibited growth by more than 65% whereas compounds 7 and 8 resulted in less than 12% inhibition. These findings were supported by a morphological examination of red cells sampled from the cultures. Cultures that had been exposed to this first group of eight chelators contained degenerate parasites that showed no evidence of nuclear division,

whereas those exposed to the other two chelators could readily be recognized as pigmented trophozoites and early schizonts.

(B) The effect of using different dosage levels than the 100 μmol/l level used in (A) was studied employing the same procedure but using four dosage levels of 10 μmol/l, 25 μmol/l, 50 μmol/l and 100 μg/l. Only the eight chelators which produced an inhibitory effect at 100 μmol/l were studied and controls containing no iron chelator were again employed. It was found that the $ID_{50}$ for the 3-hydroxypyridin-4-one compounds 1 to 6 and 9 was in the range of 25-40 μmol/l whereas for compound 10, desferrioxamine, it was in the range of 10-20 μmol/l.

A study was made of red cells from cultures involving various dosage levels harvested at the beginning of the second parasite cycle, when young rings could be identified in untreated control cultures in order to determine whether or not red cell reinvasion had occurred in the cultures that had been exposed to a chelator. No evidence was found of reinvasion following exposure to the compound 10, desferrioxamine, at a 25 μmol/l, 50 μmol/l or 100 μmol/l dosage level but it was always found at the 10 μmol/l dosage level. However, for the 3-hydroxypyridin-4-one compounds 1 to 6 and 9, young ring parasites were frequently found after incubation at a 25 μmol/l dosage level of the chelator and were always found at a 10 μmol/l dosage level, no significant differences being seen between the different compounds. At 50 μmol/l and 100 μmol/l no evidence of reinvasion was found. Thus, although the 3-hydroxypyridin-4-ones do produce growth inhibition at the lower dosage levels of 10 μmol/l and 25 μmol/l, higher levels than this are required to produce a cytocidal action.

In a further study marked growth inhibition was observed at a dosage level of 0.1 μmol/l for 1-(2-carboxyethyl)-3-hydroxy-2-methylpyridin-4-one. In the case of 2-carboxymethyl-3-hydroxy-1-methylpyridin-4-one, 1-(2-carboxyethyl)-2-ethyl-3-hydroxypyridin-4-one and 1-(3-carboxypropyl)-2-methyl-3-hydroxypyridin-4-one a dosage level of 0.5 μmol/l was required to produce a similar level of growth inhibition and in the case of 3-hydroxy-2-methyl-1-(2-sulphoethyl)pyridin-4-one a dosage level of 1 μmol/l was required.

(C) A culture of ring parasites obtained as in (A) was dispensed in 3 ml volumes into separate flasks. Different cultures were exposed during a 6 hour period to the compound 1 in a 100 μmol/l iron binding concentration at different stages during the first parasite cycle and controls containing no chelator were again employed. At the end of each 6 hour pulse, all cultures were washed twice in RPMI 1640 and the pellet was resuspended in complete medium. At 40 hours, when cells from cultures that had not been exposed at any time to a chelator contained segmenting schizonts, tritiated hypoxanthine was added to the incubation medium of all the cultures. Growth of the parasites was assessed after further periods of 42 hours (during second cycle), 65 hours (during third cycle) and 84 hours (during fourth cycle). Each pulse of compound 1 was duplicated in separate flasks. Morphologically, synchrony of the cultures was clearly lost during the third parasite cycle.

It was found that exposure of the parasites to compound 1 during the ring and trophozoite stages of parasite development resulted in minimal growth inhibition. Parasites that had been incubated with compound 1 between 6-12, 12-18 and 18-24 hours during the first cycle showed respectively 0%, 11.4% and 0% growth inhibition, during the second cycle, and respectively 0%, 12% and 0% growth inhibition during the third cycle. Furthermore, no morphological differences were detected between parasites that were sampled from these cultures and untreated parasites. On the other hand, incubation with compound 1 during the schizont stage (24-30 hours) of parasite development did produce an inhibitory effect on the growth of P. falciparum resulting in 38% and 30% growth inhibition during the second and third cycles. Morphologically these cultures contained a mixed population of degenerate forms and young ring parasites with evidence of red cell reinvasion. Thus it will be seen that the effect of compound 1 is maximised during the early schizont stage of the development of the parasite. However, the employment of 6 hour pulses in order to compare the effects of the compound at different stages, as opposed to the continuous treatment employed in (A), did lead to a lower level of anti-malarial effect.

Example 3 : Comparison of the inhibition of the growth of P.falciparum and of murine erythroleukaemia cells

MEL cells, clone 585, were grown in suspension culture at 37°C in RPMI 1640 that had been supplemented with 13% v/v foetal calf serum, 25 μl/ml gentamicin, 1.9 mM glutamine and 2% diaminopurine as described by Peto et al in Br. J. Haematol., 1983, 54, 623. The cultures were maintained in an atmosphere containing 5% carbon dioxide and passaged every 3-4 days at a seeding concentration of 1-2 x $10^4$/ml to ensure that the cells were continually in logarithmic growth phase. Under these conditions, the doubling time for cell growth was 30-36 hours.

10 ml volumes of cell suspension were incubated in 50 ml flasks with each of the ten chelators identified in the Table at a 50 μmol/l iron binding concentration, controls being employed which contained no chelator. 400 μl aliquots were removed in duplicate for growth assessment at 24 hour intervals over the next four days. The experiment was repeated on three separate occasions, the initial cell concentration in each case being 2-3 x $10^4$ cells/ml. Cell growth was assessed both by examination of cultures using light microscopy and by cell counting. At various times throughout each experiment, 400 μl culture was added to 19.6 ml Isoton and

the number of particles counted using a Coulter Counter ZN set at threshold 20 and attenuation 0.4

The effect of the different chelators on growth of the MEL cells after 30 hours was compared with the effect produced after a similar time by the same chelators at a 50 μmol/l iron binding concentration using the procedure described in Example 2(A). The results are illustrated in Figure 2. It will be seen that the compounds 2, 3, 4, 5, 6, 9 and 10 inhibited growth of MEL cells and of P. falciparum, the minimum growth inhibition being 80% and 70% respectively. The compounds 7 and 8 had little inhibitory effect on the growth of either MEL cells or P. falciparum. When these two groups of chelators were examined together a close linear correlation (r = 0.978) was found between the degree of growth inhibition produced in both types of cell for each chelator studied. The compound 1 was distinguished from all of the other chelators in being the only one to have no significant inhibitory effect on the growth of MEL cells (<10%) whilst at the same time producing a substantial inhibitory effect on the growth of P. falciparum (>75%). This pattern of growth inhibition for the ten chelators was exhibited in three separate experiments.

## Claims

1. The use for the manufacture of a medicament for use in the treatment of a condition caused by an iron dependent parasite of a 3-hydroxypyridin-4-one of formula (I)

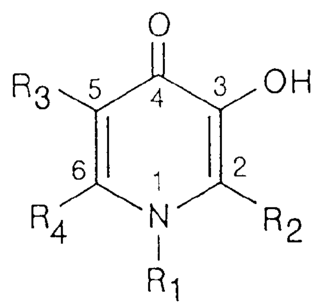

in which $R_1$ is selected from hydrogen, $C_{1-3}$ aliphatic hydrocarbon groups, 2-hydroxyethyl, and $C_{1-4}$ aliphatic hydrocarbon groups substituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, and $R_2$, $R_3$ and $R_4$ are each separately selected from hydrogen, $C_{1-3}$ aliphatic hydrocarbon groups, hydroxymethyl, 1-hydroxyethyl, 2-hydroxyethyl, methoxymethyl, $C_{1-4}$ aliphatic hydrocarbon groups substituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, but with the provisos firstly that one of $R_1$ to $R_4$ is a $C_{1-4}$ aliphatic hydrocarbon group substituted by a single carboxy, sulpho, sulphamoyl or N-methyl or N-ethyl sulphamoyl group, or an ethyl group substituted at the 1- or 2-position by a carboxy, sulpho, sulphamoyl or N-methyl or N-ethyl sulphamoyl group and at the 2-position by a hydroxy group, secondly that either $R_2$ or $R_3$ is a group other than hydrogen and thirdly that the total number of atoms other than hydrogen present in $R_1$ to $R_4$ is no more than eight, the compound optionally being in the form of a physiologically acceptable salt and/or pro-drug thereof.

2. The use according to Claim 1, in which $R_1$ is selected from methyl, ethyl and a carboxy-substituted methyl, ethyl, isopropyl or propyl group, and $R_2$, $R_3$ and $R_4$ are each separately selected from hydrogen, methyl, ethyl and a carboxy-substituted methyl, ethyl, isopropyl or propyl group.

3. The use according to Claim 1 or 2, in which $R_1$ is the group containing a carboxy, sulpho, sulphamoyl or N-methyl or N-ethyl sulphamoyl group substituent.

4. The use according to any of Claims 1 to 3, in which the total number of carbon, nitrogen, oxygen and sulphur atoms in $R_1$ to $R_4$ is five, six or seven.

5. The use according to any of the preceding claims, in which $R_3$ and $R_4$ are each hydrogen.

6. The use according to any of the preceding claims, in which the substituents in the 3-hydroxypyridin-4-one consist of one methyl or ethyl group and one carboxymethyl, 2-carboxyethyl or 3-carboxypropyl group.

7. The use according to Claim 1, in which the compound of formula (I) is 3-hydroxy-2-methyl-1-(2-sulphamoylethyl)pyridin-4-one, 3-hydroxy-2-methyl-1-(2-N-methylsulphamoylethyl)pyridin-4-one or 1-(2-N-ethylsulphamoylethyl)-3-hydroxy-2-methylpyridin-4-one.

8. The use according to Claim 1, in which the compound of formula (I) is 3-hydroxy-2-methyl-1-(2-sulphamoylethyl)pyridin-4-one.

9. The use according to Claim 1, in which the compound of formula (I) is 1-carboxymethyl-3-hydroxy-2-methylpyridin-4-one, 1-(2-carboxyethyl)-3-hydroxy-2-methylpyridin-4-one, 1-(2-carboxyethyl)-2-ethyl-3-hydroxypyridin-4-one, or 1-(3-carboxypropyl)-2-methyl-3-hydroxypyridin-4-one.

10. The use according to Claim 1, in which the compound of formula (I) is 1-(2-carboxyethyl)-3-hydroxy-2-methylpyridin-4-one

11. The use according to any of the preceding claims, in which the medicament is for use in the treatment of malaria.

12. A 3-hydroxypyridin-4-one of formula (I) as defined in Claim 1 but excluding the specific compounds 1-carboxymethyl-3-hydroxypyridin-4-one, 1-(2-carboxyethyl)-3-hydroxypyridin-4-one, 1-carboxymethyl-3-hydroxy-2-methylpyridin-4-one and 1-(2-carboxyethyl)-3-hydroxy-2-methylpyridin-4-one.

13. A compound according to Claim 12 being 3-hydroxy-2-methyl-1-(2-sulphamoylethyl)pyridin-4-one, 3-hydroxy-2-methyl-1-(2-N-methylsulphamoylethyl)pyridin-4-one or 1-(2-N-ethylsulphamoylethy1)-3-hydroxy-2-methylpyridin-4-one.

14. A compound according to Claim 12 being 1-(2-carboxyethyl)-2-ethyl-3-hydroxypyridin-4-one or 1-(3-carboxypropyl)-2-methyl-3-hydroxypyridin-4-one.

15. A compound according to any of Claims 12 to 14 for use in therapy.

**Claims for the following Contracting States : GR, ES**

1. The use for the manufacture of a medicament for use in the treatment of a condition caused by an iron dependent parasite of a 3-hydroxypyridin-4-one of formula (I)

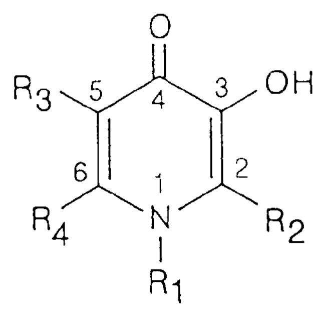

in which $R_1$ is selected from hydrogen, $C_{1-3}$ aliphatic hydrocarbon groups, 2-hydroxyethyl, and $C_{1-4}$ aliphatic hydrocarbon groups substituted by a single carboxy, sulpho, sulphamoyl or N-methyl or N-ethyl sulphamoyl group, and an ethyl group substituted at the 1- or 2-position by a carboxy, sulpho, sulphamoyl or N-methyl or N-ethyl sulphamoyl group and at the 2-position by a hydroxy group, and $R_2$, $R_3$ and $R_4$ are each separately selected from hydrogen, $C_{1-3}$ aliphatic hydrocarbon groups, hydroxymethyl, 1-hydroxyethyl, 2-hydroxyethyl, methoxymethyl, $C_{1-4}$ aliphatic hydrocarbon groups subtituted by a single carboxy, sulpho, sulphamoyl or N-methyl or N-ethyl sulphamoyl group, and an ethyl group substituted at the 1-or 2-position by a carboxy, sulpho, sulphamoyl or N-methyl or N-ethyl sulphamoyl group and at the 2-position by a hydroxy group, but with the provisos firstly that one of $R_1$ to $R_4$ is a $C_{1-4}$ aliphatic hydrocarbon group substituted by a single carboxy, sulpho, sulphamoyl or N-methyl or N-ethyl sulphamoyl group, or an ethyl group substituted at the 1- or 2-position by a carboxy, sulpho, sulphamoyl or N-methyl or N-ethyl sulphamoyl group and at the 2-position by a hydroxy group, secondly that either $R_2$ or $R_3$ is a group other than hydrogen and thirdly that the total number of atoms other than hydrogen present in $R_1$ to $R_4$ is no more than eight, the compound optionally being in the form of a physiologically acceptable salt and/or pro-drug thereof.

**2.** The use according to Claim 1, in which $R_1$ is selected from methyl, ethyl and a carboxy-substituted methyl, ethyl, isopropyl or propyl group, and $R_2$, $R_3$ and $R_4$ are each separately selected from hydrogen, methyl, ethyl and a carboxy-substituted methyl, ethyl, isopropyl or propyl group.

**3.** The use according to Claim 1 or 2, in which $R_1$ is the group containing a carboxy, sulpho, sulphamoyl or N-methyl or N-ethyl sulphamoyl group substituent.

**4.** The use according to any of Claims 1 to 3, in which the total number of carbon, nitrogen, oxygen and sulphur atoms in $R_1$ to $R_4$ is five, six or seven.

**5.** The use according to any of the preceding claims, in which $R_3$ and $R_4$ are each hydrogen.

**6.** The use according to any of the preceding claims, in which the substituents in the 3-hydroxypyridin-4-one consist of one methyl or ethyl group and one carboxymethyl, 2-carboxyethyl or 3-carboxypropyl group.

**7.** The use according to Claim 1, in which the compound of formula (I) is 3-hydroxy-2-methyl-1-(2-sulphamoylethyl)pyridin-4-one, 3-hydroxy-2-methyl-1-(2-N-methylsulphamoylethyl)pyridin-4-one or 1-(2-N-ethylsulphamoylethyl)-3-hydroxy-2-methylpyridin-4-one.

**8.** The use according to Claim 1, in which the compound of formula (I) is 3-hydroxy-2-methyl-1-(2-sulphamoylethyl)pyridin-4-one.

**9.** The use according to Claim 1, in which the compound of formula (I) is 1-carboxymethyl-3-hydroxy-2-methylpyridin-4-one, 1-(2-carboxyethyl)-3-hydroxy-2-methylpyridin-4-one, 1-(2-carboxyethyl)-2-ethyl-3-hydroxypyridin-4-one, or 1-(3-carboxypropyl)-2-methyl-3-hydroxypyridin-4-one.

**10.** The use according to Claim 1, in which the compound of formula (I) is 1-(2-carboxyethyl)-3-hydroxy-2-methylpyridin-4-one

**11.** The use according to any of the preceding claims, in which the medicament is for use in the treatment of malaria.

**12.** A process for the preparation of a 3-hydroxypyridin-4-one of formula (I) as defined in Claim 1 but excluding the specific compounds 1-carboxymethyl-3-hydroxypyridin-4-one, 1-(2-carboxyethyl)-3-hydroxypyridin-4-one, 1-carboxymethyl-3-hydroxy-2-methylpyridin-4-one and 1-(2-carboxyethyl)-3-hydroxy-2-methylpyridin-4-one, which comprises:

(a) reacting a 3-hydroxy-4-pyrone of formula (VI)

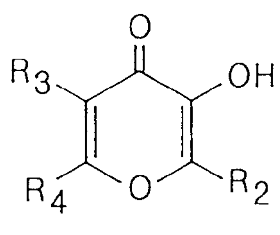

(VI)

in which $R_2$ to $R_4$ correspond to the same groups as are present in the compound of formula (I) or are

groups convertible thereto, with a compound $R'NH_2$ in which R′ corresponds to the group $R_1$ in the compound of formula (I) or is a group convertible thereto;
(b) reacting a 3-hydroxypyridin-4-one of formula (VII)

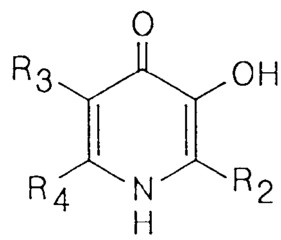

(VII)

in which $R_2$ to $R_4$ correspond to the same groups as are present in the compound of formula (I) or are groups convertible thereto, with a compound R′X in which R′ corresponds to the group $R_1$ in the compound of formula (I) or is a group convertible thereto;
(c) reacting a 3-hydroxypyrid-4-one of formula (VII) as defined in (b) with a compound $R'NH_2$ in which R′ corresponds to the group $R_1$ in the compound of formula (I) or is a group convertible thereto; or
(d) treating a protected 3-hydroxypyridin-4-one of formula (VIII)

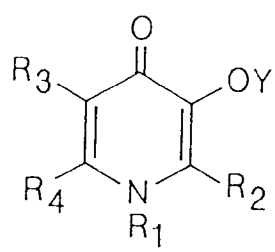

(VIII)

in which $R_1$ to $R_4$ correspond to the same groups as are present in the compound of formula (I) or are groups convertible thereto and Y is a hydroxy protecting group, to remove the protecting group Y and generate the hydroxy group; procedure (d) optionally being combined with one of procedures (a), (b) and (c) through the use of a compound of formula (VI) or (VII) in that procedure in which the hydroxy group thereof is in protected form as a group OY; and, where appropriate, in any procedure or combination of procedures, effecting the conversion of one or more of the groups $R_1$ to $R_4$ in the product to those present in the compound of formula (I) and/or converting the product to salt and/or pro-drug form.

**13.** A process according to Claim 12, in which procedure (d) is used.

**14.** A process according to Claim 12 or 13, in which the compound of formula (I) is 3-hydroxy-2-methyl-1-(2-sulphamoylethyl)pyridin-4-one, 3-hydroxy-2-methyl-1-(2-N-methylsulphamoylethyl) pyridin-4-one or 1-(2-N-ethylsulphamoylethyl)-3-hydroxy-2-methylpyridin-4-one.

**15.** A process according to Claim 12 or 13, in which the compound of formula (I) is 1-(2-carboxyethyl)-2-ethyl-3-hydroxypyridin-4-one or 1-(3-carboxypropyl)-2-methyl-3-hydroxypyridin-4-one.

**16.** A compound of formula (I) as defined in any of Claims 12, 14 and 15 for use in therapy.

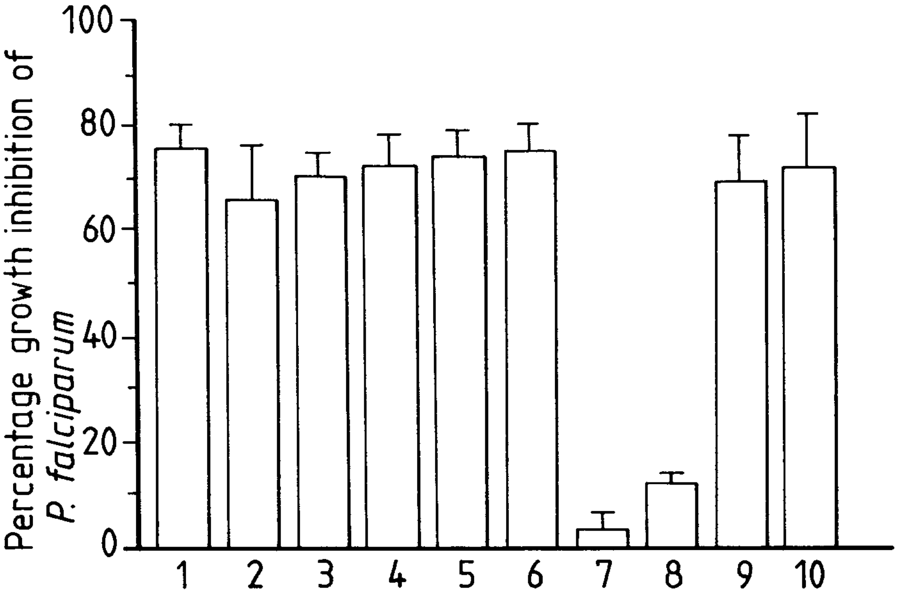
Percentage growth inhibition of
P. falciparum
100
80
60
40
20
0
1
2
3
4
5
6
7
8
9
10

Fig.1

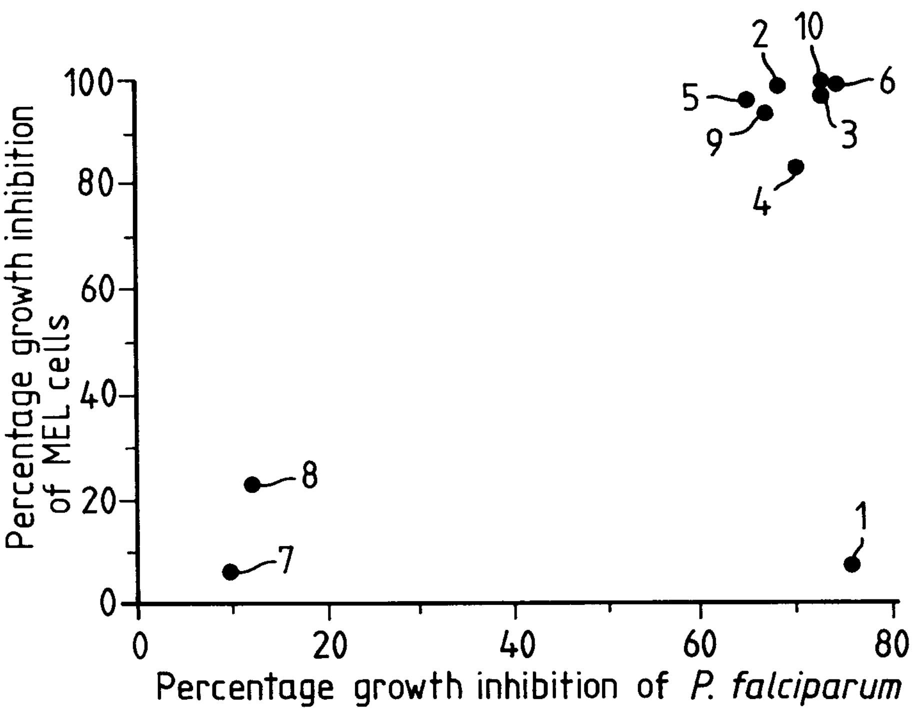
Percentage growth inhibition
of MEL cells
100
80
60
40
20
0
0
20
40
60
80
Percentage growth inhibition of P. falciparum
1
2
3
4
5
6
7
8
9
10

Fig.2

European Patent Office

# EUROPEAN SEARCH REPORT

Application Number

EP 92 30 0120

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | JOURNAL OF INORGANIC BIOCHEMISTRY, vol. 35, no. 1, 1989, pages 9-22; M.C. BRADY et al.: "Release of iron from ferritin molecules and their iron-cores by 3-hydroxypyridinone chelators in vitro" * Page 9; page 10, compound CP38 * --- | 1-6,9-10 | C 07 D 213/69<br>A 61 K 31/44 |
| A,D | CHEMICAL ABSTRACTS, vol. 110, 1989, page 18, abstract no. 18109s, Columbus, Ohio, US; J.B. PORTER et al.: "Iron mobilization from hepatocyte monolayer cultures by chelators: the importance of membrane permeability and the iron-binding constant", & BLOOD 1988, 72(5), 1497-503 * Abstract * --- | | |
| X | EP-A-0 120 669 (NATIONAL RESEARCH DEVELOPMENT) * Examples 16,17; pages 1,2; claim 12 * --- | 1-6,9 | |
| A,D | EP-A-0 316 279 (CIBA-GEIGY) * Page 4, lines 44-47 * --- | 1,11,15 | **TECHNICAL FIELDS SEARCHED (Int. Cl.5)** |
| A | EP-A-0 305 646 (NATIONAL RESEARCH DEVELOPMENT) * Page 6, formula VI; page 7; claims * ----- | 1-6 | C 07 D 213/00<br>A 61 K 31/00 |

The present search report has been drawn up for all claims

| Place of search | Date of completion of the search | Examiner |
|---|---|---|
| THE HAGUE | 10-03-1992 | DE JONG B.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)